# EUROPEAN PATENT APPLICATION

(11) **EP 3 128 013 A1**
(43) Date of publication of application: **08.02.2017**
(21) Application number: 15382419.8
(22) Date of filing: 07.08.2015
(51) Int. Cl.: C12P 19/30, C12P 9/00, C12P 17/16

(54) **DEAMINATION OF ORGANOPHOSPHOROUS-NUCLEOSIDES**

(71) Applicant: Institut Univ. de Ciència i Tecnologia, S.A., 08100 Mollet del Vallès Barcelona (ES)
(72) Inventor: PÉREZ OZCÁRIZ, Sergio, 08100 MOLLET DEL VALLES (ES); PASCUAL GILABERT, Marta, 08100 MOLLET DEL VALLÈS (ES); FERNÁNDEZ FERNÁNDEZ, Carmen María, 08100 MOLLET DEL VALLES (ES); CASTELLS BOLIART, Josep, 08100 MOLLET DEL VALLES (ES)
(74) Representative: Oficina Ponti, SLP

(57) **Abstract**

The invention relates to a new synthethic process for obtaining compounds of formula (I) from compounds of formula (II) by means of deaminase enzymes.

## Description

### Field of the invention

The present invention relates to a novel enzymatic process for nucleoside deamination, in particular, for the deamination of organophosphorous nucleoside analogues (NAs), and more in particular for the deamination of cytosinic organophosphorous NAs as well as drugs, intermediates or prodrugs thereof.

### Background of the invention

Nucleoside analogues (NAs) are synthetic compounds structurally related to natural nucleosides. In terms of their structure, nucleosides are constituted by three key elements: (i) the hydroxymethyl group, (ii) the heterocyclic nitrogenous base moiety, and (iii) the furanose ring, which in several instances seems to act as a spacer presenting the hydroxymethyl group and the base in the correct orientation.

NAs are extensively used as antiviral and antitumor agents. These molecules have been traditionally synthesized by different chemical methods which often require time-consuming multistep processes including protection-deprotection reactions on the heterocycle base and/or the pentose moiety to allow the modification of naturally occurring nucleosides (Boryski J. 2008. Reactions of transglycosylation in the nucleoside chemistry. Curr Org Chem 12:309-325). This time consuming multistep processes often lead to low yields and increased costs. Indeed, chemical methods usually increase the difficulty of obtaining products with correct stereo- and regioselectivity, generating by-products as impurities (Condezo, L. A., et al. 2007. Enzymatic synthesis of modified nucleosides, p. 401-423. Biocatalysis in the pharmaceutical and biotechnology industries. CRC Press, Boca Raton, FL, Mikhailopulo, I. A. 2007; Sinisterra, J.V. et al. 2010. Enzyme-catalyzed synthesis of nonnatural or modified nucleosides, p. 1-25. Encyclopedia of Industrial Biotechnology: Bioprocess, Bioseparation, and Cell Technology, John Wiley & sons, Ed. By M. C. Flickinger, 2010). Moreover, the chemical methods include the use of chemical reagents and organic solvents that are expensive and environmentally harmful.

Therefore, enzymatic approaches have special interest because they can solve some of these problems. In particular, the deamination of amino-containing nucleosides is an interesting way to synthesize their corresponding keto-counterparts. Deamination reactions occurring in natural nucleosides, either ribo- or 2'-deoxyribonucleosides, take place at the nucleobase moiety, including cytosine, 5-methylcytosine, guanine and adenine nucleosides, that are transformed into their corresponding nucleoside analogues containing, respectively, uracil, thymine, xanthine and hypoxanthine as the nucleobases, and ammonia as by-product.

Although deaminase enzymes are broadly distributed, usually they are very specific for their corresponding substrates (Katsiragi, T. et al. 1986. Cytosine Deaminase from Escherichia coli - Production, Purification, and Some characteristics, Agric. Biol. Chem. 50(7), 1721-1730; Vita, A. et al. 1985. Cytidine Deaminase from Eschericia coli B. Purification and Enzymatic Molecular Properties, Biochemistry, 24, 6020-6024).

According to this specificity, deaminases can be divided into: nucleobase deaminases (such as cytosine deaminase, EC 3.5.4.1, *i.e.* natural substrate is the nucleobase itself), nucleoside deaminases (such as cytidine deaminase, EC 3.5.4.5, *i.e.* natural substrate is the nucleoside) and nucleotide deaminases (such as 2'-deoxycytidine triphosphate deaminase, EC 3.5.4.13, *i.e.* natural substrate is the nucleotide).

Accordingly, nucleoside deaminases are able to deaminate nucleosides but not nucleotides, whereas nucleotide deaminases are able to deaminate nucleotides but not nucleosides.

Taking the structure of the substrates into consideration, organophosphorous nucleosides, *i.e.* those nucleosides bearing a substituted phosphor atom connected to the oxygen at nucleosidic position C-5', such as organic phosphates, phosphinates, phosphonates, phosphoramidates, and the like, should exhibit a substrate behavior and specificity similar to natural nucleotides (i.e. a nucleoside plus a phosphate group). Therefore, for those skilled in the art, the enzymes of choice for catalyzing their corresponding deamination would be nucleotide deaminases.

Furthermore, the deamination of certain nucleosidic substrates incorporating bulky substituents remains an unresolved problem because of their difficult fitting into the active site of the enzymes. In particular, those NA containing mono-, di- or triphosphate groups bounded to the sugar ring are known to usually act as inhibitors of these enzymes (Faivre-Nitschke, S.E. et al. 1999, A prokaryotic-type cytidine deaminase from Arabidopsis thaliana, Eur. J. Biochem. 263, 896-903).

Surprisingly, it was found that the drawbacks of previous cited biocatalytic synthesis on organophosphorous nucleosides can be avoided by applying an enzymatic method based on the use of nucleoside deaminase enzymes. The referred enzymes, surprisingly, can recognize proper modified phosphour-containing nucleoside analogs substrates and are able to perform the deamination reaction in spite of bearing bulky substituents at position C-5'. The inventors have demonstrated that the same biocatalytic reaction but using nucleotides as substrates does not render the corresponding deaminated product.

### Description of the invention

There present invention relates to a process for preparing a compound of formula (I) according to the following reaction catalyzed by a nucleoside deaminase, preferably a cytidine deaminase (hereinafter, simply referred as Reaction II-I) 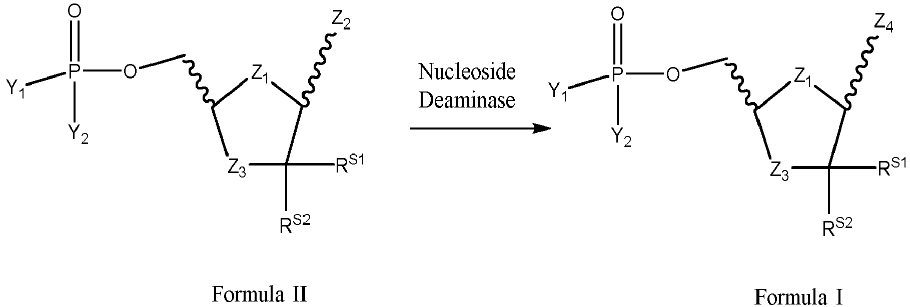 wherein
Z₁ is selected from O, CH₂, S and NH;
Z₃ is selected, independently of Z₁, from O, C(R^{S3}R^{S4}), S(^{RS3}R^{S4}), S(R^{S3}) and N(R^{S3});
Z₂ is selected from: 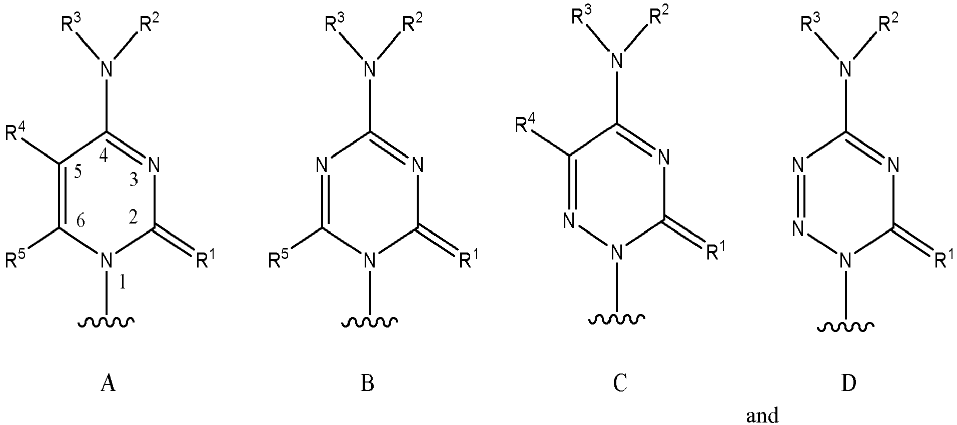
Z₄ is selected from: 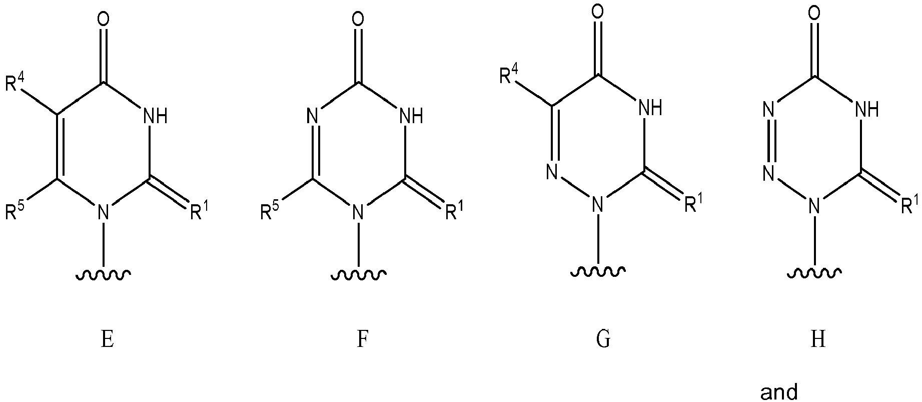
R¹ is selected from O, CH₂, alkyl, S and NH;
R² is selected from hydrogen; methyl; an optionally substituted alkyl chain; an optionally substituted alkenyl chain; an optionally substituted alkynyl chain; trihaloalkyl; NR⁶R⁷; CN; COR⁶; CONR⁶R⁷; CO₂R⁶; C(S)R⁶; C(S)OR⁶; NHCONR⁶R⁷; NHCO₂R⁶; NHC(S)OR⁶; SO₂R⁶; SO₂NR⁶R⁷; an optionally substituted aryl linked to N-4 by an optionally substituted alkyl, alkenyl or alkynyl chain; and an optionally substituted heterocycle linked to N-4 by an optionally substituted alkyl, alkenyl or alkynyl chain;
R³ is selected, independently of R², from hydrogen; methyl; an optionally substituted alkyl chain; an optionally substituted alkenyl chain; an optionally substituted alkynyl chain; trihaloalkyl; NR⁶R⁷; CN; COR⁶; CONR⁶R⁷; CO₂R⁶; C(S)R⁶; C(S)OR⁶; NHCONR⁶R⁷; NHCO₂R⁶; NHC(S)OR⁶; SO₂R⁶; SO₂NR⁶R⁷; an optionally substituted aryl linked to N-4 by an optionally substituted alkyl, alkenyl or alkynyl chain; and an optionally substituted heterocycle linked to N-4 by an optionally substituted alkyl, alkenyl or alkynyl chain;
providing that at least one of R² and R³ is H;
R⁴ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶;NHCONR⁶R⁷; NHCOR⁶; NR⁶CO₂R⁷; NHCO₂R⁶; NHC(S)OR⁶; SO₂NR⁶R⁷; an optionally substituted aryl linked to C-5 by an optionally substituted alkyl, alkenyl or alkynyl chain; and an optionally substituted heterocycle linked to C-5 by an optionally substituted alkyl, alkenyl or alkynyl chain;
R⁵ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-6 by an optionally substituted alkyl, alkenyl or alkynyl chain; and an optionally substituted heterocycle linked to C-6 by an optionally substituted alkyl, alkenyl or alkynyl chain;
R⁶ and R⁷ are selected, independently of each other, from hydrogen; an optionally substituted alkyl chain; an optionally substituted alkenyl chain; an optionally substituted alkynyl chain; an optionally substituted heterocycle; and an optionally substituted aryl, preferably phenyl or naphtyl;
R^{S1} is selected, independently of R^{S2}, from hydrogen; halogen, preferably F; methyl; OH; NH₂; SH; N₃; an optionally substituted alkyl chain; an optionally substituted alkenyl chain; an optionally substituted alkynyl chain; trihaloalkyl, OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S2} is selected, independently of R^{S1}, from hydrogen; halogen, preferably F; methyl; OH; NH₂; SH; N₃; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S3} is selected, independently of R^{S4}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; and an optionally substituted aryl; an optionally substituted heterocycle; OR⁶; OCONR⁶R⁷; OCOR⁶; OCO₂R⁶; and OC(S)OR⁶;
R^{S4} is selected, independently of R^{S3}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; an optionally substituted aryl; an optionally substituted heterocycle; OR⁶; OCONR⁶R⁷; OCOR⁶; OCO₂R⁶; and OC(S)OR⁶;
Y₁ is selected, independently of Y₂, from hydrogen; OR⁸; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O-*naphtyl; an ether of an optionally substituted heterocycle; and an amino acid, preferably alanine, valine, leucine or isoleucine, either in the free form or protected by a suitable functional group;
Y₂ is selected, independently of Y₁, from hydrogen; OH; OR⁸; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O-*naphtyl; an ether of an optionally substituted heterocycle; an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; and an optionally substituted heterocycle optionally linked to P through O or N atoms; aryl, preferably phenyl or naphthyl;
and wherein the compound of formula (I) or formula (II) can also be a derivative thereof, being said derivative an acceptable pharmaceutically salt thereof; an ester, amide, carbamate or phosphate thereof; an addition salt thereof; a quaternary ammonium salt thereof; or an N-oxide thereof.

Applicants have surprisingly found that cytosine containing organophosphorous-nucleoside analogues, represented by formula II, are recognized as substrates by nucleoside deaminase at a conversion rate equivalent to their natural substrates, i.e. nucleoside analogues, instead of being recognized as nucleotide analogues, which are, in fact, nonreactive under the same reaction conditions.

Accordingly, cytosine containing organophosphorous-nucleoside analogues described herein allow the preparation/production of uridinic nucleoside analogues at high conversions (more than 70%, usually quantitative), and at the same time high specificity, completely avoiding side reactions, such as the deamination at non desired positions.

No evidences have been found so far pointing at the fact that this sort of chemical modification at position C-5' in cytidine derivatives chemical backbone, would have been able to modify the substrate specificity for nucleoside deaminases.

In the context of the present invention, the term uridine or uridinic derivatives, nucleosides, intermediates, they all should be understood as chemical compounds derived from uridine backbone. In particular, the uridine or uridinic derivatives are uridine containing organophosphorous-nucleoside analogues, represented by formula (I).

In the context of the present invention, the term cytidine or cytidinic derivatives, nucleosides, intermediates, they all should be understood as chemical compounds derived from cytidine backbone. In particular, cytidine or cytidinic derivatives are cytosine containing organophosphorous-nucleoside analogues, represented by formula (II).

In a preferred embodiment for the process for preparing a compound of formula (I) as defined above in Reaction II-I:
Z₁ is selected from O and CH₂, more preferably O;
Z₃ is selected, independently of Z₁, from O and C(R^{S3}R^{S4}), more preferably C(R^{S3}R^{S4});
Z₂ is selected from 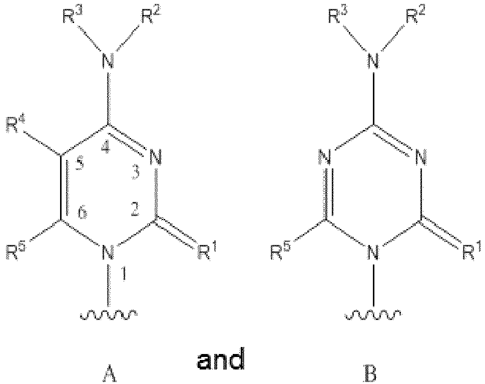
R¹ is O;
R² is selected from H; methyl; an optionally substituted alkyl chain; COR⁶, CONR⁶R⁷, CO₂R⁶, and C(S)OR⁶; more preferably selected from H; COR⁶; CONR⁶R⁷; CO₂R⁶; and even more preferably R² is COR⁶, CONR⁶R⁷, or CO₂R⁶;
R³ is selected, independently of R², from H; methyl; an optionally substituted alkyl chain; COR⁶; CONR⁶R⁷; CO₂R⁶; C(S)OR⁶; more preferably R³ is H;
providing that at least one of R² and R³ is H;
R⁴ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R⁵ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F, methyl; OH; OR⁶; NR⁶R⁷; OCONR⁶R⁷; OCOR⁶; OCO₂R⁶; and OC(S)OR⁶;
R^{S2} is selected, independently of R^{S1}, from hydrogen; halogen, preferably F, methyl; OH; OR⁶; NR⁶R⁷; OCONR⁶R⁷; OCOR⁶; OCO₂R⁶; and OC(S)OR⁶;
R^{S3} is selected, independently of R^{S4}, from hydrogen; OH; OR⁶; OCONR⁶R⁷; OCOR⁶; OCO₂R⁶; OC(S)OR⁶; and halogen, preferably F;
R^{S4} is selected, independently of R^{S3}, from hydrogen; OH; OR⁶; OCONR⁶R⁷; OCOR⁶; OCO₂R⁶; OC(S)OR⁶; and halogen, preferably F;
Y₁ is selected, independently of Y₂, from hydrogen; OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from hydrogen; OH; OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

In a more preferred embodiment for the process for preparing a compound of formula (I) as defined above in Reaction II-I:
Z₁ is O;
Z₃ is C(R^{S3}R^{S4};
Z₂ is selected from 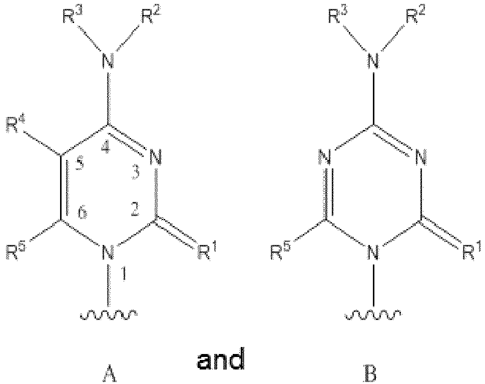
R¹ is O;
R² is selected from H; methyl; COR⁶; CONR⁶R⁷; and CO₂R⁶;
R³ is selected, independently of R², from H; methyl; COR⁶; CONR⁶R⁷; CO₂R⁶; and C(S)OR⁶; more preferably R³ is COR⁶, CONR⁶R⁷ or CO₂R⁶;
providing that at least one of R² and R³ is H;
R⁴ is selected from H; OH; halogen, preferably F; and trihaloalkyl;
R⁵ is selected from H; OH; halogen; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F; methyl; OH; OR⁶; OCONR⁶R⁷; OCOR⁶; and OCO₂R⁶;
R^{S2} is selected, independently of R^{S1}, from hydrogen; halogen preferably F; methyl; OH; OR⁶; OCONR⁶R⁷; OCOR⁶; OCO₂R⁶;
R^{S3} is selected, independently of R^{S4}, from hydrogen; OH; OR⁶; OCONR⁶R⁷; OCOR⁶; OCO₂R⁶; OC(S)OR⁶; halogen, preferably F;
R^{S4} is selected, independently of R^{S3}, from hydrogen; OH; OR⁶; OCONR⁶R⁷; OCOR⁶; OCO₂R⁶; OC(S)OR⁶; halogen, preferably F;
Y₁ is selected, independently of Y₂, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from hydrogen; OH; OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

In an even more preferred embodiment for the process for preparing a compound of formula (I) as defined above in Reaction II-I:
Z₁ is O;
Z₃ is C(R^{S3}R^{S4}) wherein R^{S3} is H or OH and wherein R^{S4} is, independently of R^{S3}, H or OH; Z₂ is 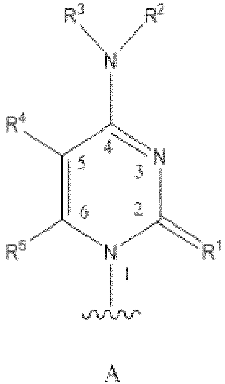
R¹ is O;
R² is H;
R³ is H;
R⁴ is H or halogen, preferably F;
R⁵ is H;
R^{S1} is selected, independently of R^{S2}, from hydrogen; halogen, preferably F; methyl; and OH; R^{S2} is selected, independently of R^{S1}, from hydrogen; halogen, preferably F; methyl; and OH; Y₁ is selected, independently of Y₂, from OR⁸; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; NHCONR⁶R⁷; NHCOR⁶; NR⁶CO₂R⁷; NHCO₂R⁶; NHC(S)OR⁶; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

In an even more preferred embodiment for the process for preparing a compound of formula (I) as defined above in Reaction II-I,
Z₁ is O;
Z₃ is C(R^{S3}R^{S4}) wherein R^{S3} is H or OH and wherein R^{S4} is, independently of R^{S3}, H or OH;
Z₂ is 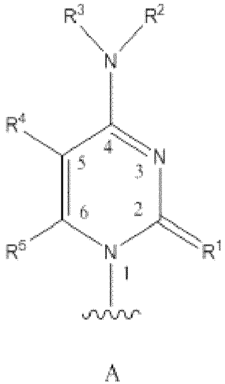
R¹ is O;
R² is H;
R³ is H;
R⁴ is H or halogen, preferably F;
R⁵ is H;
R^{S1} is selected, independently of R^{S2}, from hydrogen; halogen, preferably F; methyl; and OH;
R^{S2} is selected, independently of R^{S1}, from hydrogen; halogen, preferably F; methyl; and OH;
Y₁ is selected, independently of Y₂, from OR⁸; an ether of an optionally substituted aryl, preferably *O*-phenyl or O-naphtyl; an ether of an optionally substituted heterocycle; and an amino acid, preferably alanine, valine, leucine or isoleucine, either in the free form or protected by a suitable functional group;
more preferably Y₁ is selected, independently of Y₂, from an ether of an optionally substituted aryl, preferably *O*-phenyl; and an amino acid, preferably alanine, valine, leucine or isoleucine, either in the free form or protected by a suitable functional group;
Y₂ is selected, independently of Y₁, from an ether of an optionally substituted aryl, preferably *O*-phenyl or *O*-naphtyl; and an amino acid, preferably alanine, valine, leucine or isoleucine, either in the free form or protected by a suitable functional group;
more preferably Y₂ is selected, independently of Y₁, from an ether of an optionally substituted aryl, preferably *O*-phenyl; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

The suitable chemical modifications that are the object of the present invention provide a more convenient, efficient and easier process for the one-step production of cytidine organophosphorous nucleoside analogues bearing bulky substituent groups at position C-5' from their corresponding cytidine counterparts, that fully avoids the inhibition problems. Particularly preferred phosphor substitution is in the form of phosphoramidate derivatives, more preferably phosphoramidate groups including an aromatic group such as phenyl and an amino acid such as alanine, preferably protected at the carbon in the terminal end as isopropyl ester, being this then a suitable functional group for protection.

Hence, the invention provides improved alternative synthesis methods of nucleoside analogues, useful as anticancer and/or antiviral products, by shortening conventional multistep synthesis, increasing overall yield, reducing side reactions and by-product content and, therefore, improving product purity and quality.

For the purposes of present description, the following terms are further defined as follows.

The term "nucleoside" refers to all compounds in which a heterocyclic base is covalently coupled to a sugar, and especially preferred coupling of the nucleoside to the sugar includes a C1'-(glycosidic) bond of a carbon atom in a sugar to a carbon- or heteroatom (typically nitrogen) in the heterocyclic base. Therefore, in the present context the term "nucleoside" means the glycoside of a heterocyclic base.

The term "nucleoside" used herein is used broadly as to include, naturally occurring nucleosides and non-naturally occurring nucleosideslllustrative examples of nucleosides are ribonucleosides comprising a ribose moiety as well as deoxyribonucleosides comprising a deoxyribose moiety. With respect to the bases of such nucleosides, it should be understood that this may be any of the naturally occurring bases, e.g. adenine, guanine, cytosine, thymine, and uracil, as well as any modified variants thereof or any possible unnatural bases.

The term "nucleoside analogue", "nucleoside analog", "NA" or "NAs" as used herein refers to all nucleosides in which at least one atom of the structure is different from those present in natural nucleosides *(i.e.,* adenosine, cytidine, uridine, thymidine, inosine, guanosine, among others).

The term "organophosphorous nucleoside" refers to those nucleosides bearing a substituted phosphour atom connected to the oxygen at position C-5' and represented as compounds of formula I and compounds of formula II. The organophosphorous nucleoside analogues describe herein are intended to include, but not limited to organic phosphates, phosphinates, phosphonates, phosphoramidates, and the like, but excluding nucleotides (i.e. the substitution on OH-5' is either mono-, di- or triphosphate).

The term "nucleotide" refers to a nucleoside wherein at least one phosphate group is coupled to the sugar through oxygen at C-5' position. Natural nucleotides bear one, two or three phosphate groups.

As further used herein, the term "sugar" refers to all carbohydrates and derivatives thereof, wherein particularly contemplated derivatives include deletion, substitution or addition or a chemical group or atom in the sugar. For example, especially contemplated deletions include 2'-deoxy, 3'-deoxy, 5'-deoxy and/or 2',3'-dideoxy-sugars. Especially contemplated substitutions include replacement of the ring-oxygen with sulphur or methylene, or replacement of a hydroxyl group with a halogen, azido, amino-, cyano, sulfhydryl-, or methyl group, and especially contemplated additions include methylene phosphonate groups. Further contemplated sugars also include sugar analogues (i.e., not naturally occurring sugars), and particularly carbocyclic ring systems. The term "carbocyclic ring system" as used herein refers to any molecule in which a plurality or carbon atoms form a ring, and in especially contemplated carbocyclic ring systems the ring is formed from 3, 4, 5, or 6 carbon atoms.

The term "chemo-enzymatic synthesis" refers to a method of synthesis of chemical compounds through a combination of chemical and biocatalytic steps.

The term "enzymatic synthesis" refers to a method of synthesis of chemical compounds by means of a process which only comprises biocatalytic steps, carried out by the appropriate enzyme. Accordingly, other preferred embodiment of the synthesis process described herein is a full biocatalytic process which departs from cytosine derivatives, as the ones previously mentioned as represented by general formula II, already prepared or available in the market as cytosine derivatives as such.

For the purposes of the present application, the term "process" is intented to include both enzymatic and chemo-enzymatic synthesis, i.e. wherein at least one of the steps in the process employs an enzyme.

The terms "heterocyclic ring" or "heterocyclic base" or "base" or "nucleobase" are used interchangeably herein and refer to any compound in which plurality of atoms form a ring via a plurality of covalent bonds, wherein the ring includes at least one atom other than a carbon atom. Particularly contemplated heterocyclic bases include 5- and 6-membered rings containing at least 1 to 4 heteroatoms each independently selected from nitrogen, oxygen and sulphur as the non-carbon atom (e.g., imidazole, pyrrole, triazole, dihydropyrimidine). Further contemplated heterocycles may be fused (i.e., covalently bound) to another ring or heterocycle, and are thus termed "fused heterocycle" or "fused heterocyclic base" as used herein. Especially contemplated fused heterocycles include a 5-membered ring fused to a 6-membered ring (e.g., purine, pyrrolo[2,3-*d*]pyrimidine), and a 6-membered ring fused to another 6-membered or higher ring (e.g., pyrido[4,5-*d*]pyrimidine, benzodiazepine). Still further contemplated heterocyclic bases may be aromatic, or may include one or more double or triple bonds. Moreover, contemplated heterocyclic bases and fused heterocycles may further be substituted in one or more positions. And any one of the rings being optionally substituted with one, two or three substituents each independently selected from the group consisting of halogen, hydroxy, nitro, cyano, carboxyl, C₁₋₆alkyl, C₁₋₆alkoxy, C₁₋₆alkoxyC₁-₆alkyl, C₁₋₆alkylcarbonyl, amino, mono- or diC₁₋₆alkylamino, azido, mercapto, polyhaloC₁₋₆alkyl, polyhaloC₁₋₆alkoxy, and C₃₋₇cycloalkyl.

The terms "nucleobase" covers naturally occurring nucleobases as well as non-naturally occurring nucleobases. It should be clear to the person skilled in the art that various nucleobases which previously have been considered "non-naturally occurring" have subsequently found in nature. Thus, "nucleobase" includes not only the known purine and pyrimidine heterocycles, but also heterocyclic analogues (such as *N*-substituted heterocycles) and tautomers thereof. Illustrative examples of nucleobases are adenine, guanine, thymine, cytosine, uracil, purine, xanthine, 2-chloroadenine, 2-fluoroadenine, pentyl (5-fluoro-2-oxo-1,2, dihydropyrimidin-4-yl)carbamate, cytosine *N*-alkyl carbamates, cytosine *N*-alkylesters, 5-azacytosine, 5-bromovinyluracil, 5-fluorouracil, 5-trifluromethyluracil, 6-methoxy-9*H*-purin-2-amine and (*R*)-3,6,7,8-tetrahydroimidazo[4,5-*d*][1,3]diazepin-8-ol.

The term "nucleobase" is intended to cover every and all of these examples as well as analogues and tautomers, and regioisomers thereof. In order to differentiate these "nucleobases" from other heterocyclic bases also present in this specification, for the purposes of present specification, the term "nucleobase" mainly refers to cytosinic bases represented as Z² in formula II and as uridinic bases represented by Z⁴ in formula I.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

The term "regioisomer" refers to structural isomer, or constitutional isomer in the sense that refers to molecules with the same molecular formula that whose atoms are bonded in different order of connectivity.

The term "conversion" refers to is the percentage of starting material that is transformed into products, either the expected final product, byproducts, or even into products of degradation.

The term "yield" is the number of synthesized molecules of product per number of starting molecules. In a multistep synthesis, the yield can be calculated by multiplication of the yields of all the single steps.

The term "anomeric purity" refers to the amount of a particular anomer of a compound divided by the total amount of all anomers of that compound present in the mixture multiplied by 100.

The term "intermediate" or "intermediates" refer to any nucleoside analogue type compounds which may be transformed into the final product, the final product being preferably an active pharmaceutical ingredient (API) of nucleosidic structure, by means of suitable additional chemical reactions. Therefore, intermediates are molecules that may be considered as API precursors. The compounds of the present invention can also be considered as intermediate compounds and as such are also included in the scope of the present invention.

The term "prodrug" as used throughout this text means the pharmacologically acceptable derivatives such as esters, amides, carbamates and phosphates, such that the resulting *in vivo* biotransformation product of the derivative is the active drug as defined in the compounds of formula (I). The reference by Goodman and Gilman (The Pharmacological Basis of Therapeutics, 8th ed, McGraw-Hill, Int. Ed. 1992, "Biotransformation of Drugs", p 13-15) describing prodrugs generally is hereby incorporated. Prodrugs preferably have excellent aqueous solubility, increased bioavailability and are readily metabolized into the active inhibitors *in vivo.* Prodrugs of a compound of the present invention may be prepared by modifying functional groups present in the compound in such a way that the modifications are cleaved, either by routine manipulation or *in vivo,* to the parent compound.

Preferred prodrugs are pharmaceutically acceptable ester, amide and carbamate that are hydrolysable *in vivo* and are derived from those compounds of formula I having a hydroxy or an amino group. An *in vivo* hydrolysable ester, amide and carbamate is an ester, amide or carbamate group which is hydrolysed in the human or animal body to produce the parent acid or alcohol. Suitable pharmaceutically acceptable esters, amide and carbamates for amino group include C₁₋₆alkoxymethyl esters for example methoxymethyl, C₁₋₆ alkanoyloxymethyl esters for example pivaloyloxymethyl, phthalidyl esters, C₃₋₈ cycloalkoxycarbonyloxyC₁₋₆alkyl esters for example I-cyclohexylcarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyl-oxyethyl which may be formed at any carboxy group in the compounds of this invention.

An *in vivo* hydrolysable ester of a compound of the formula (I) containing a hydroxy group includes inorganic esters such as phosphate esters and α-acyloxyalkyl ethers and related compounds which as a result of the *in vivo* hydrolysis of the ester breakdown to give the parent hydroxy group. Examples of α-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4-position of the benzoyl ring.

For therapeutic use, salts of either the compounds of formula I or the compounds of formula II are those wherein the counter-ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the scope of the present invention.

The pharmaceutically acceptable acid and base addition salts as mentioned above are meant to comprise the therapeutically active non-toxic acid and base addition salt forms which either the compounds of formula I or the compounds of formula II are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

Either the compounds of formula I or the compounds of formula II containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term "addition salt" as used hereinabove also comprises the solvates which either the compounds of formula I or the compounds of formula II as well as the salts thereof, are able to form. Such solvates are for example hydrates, alcoholates and the like.

The term "quaternary amine" as used hereinbefore defines the quaternary ammonium salts which either the compounds of formula I or the compounds of formula II are able to form by reaction between a basic nitrogen of either the compounds of formula I or the compounds of formula II and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen.Pharmaceutically acceptable counterions include chloro, bromo, iodo, trifluoroacetate and acetate. The counterion of choice can be introduced using ion exchange resins.

The N-oxide forms of the present compounds are meant to comprise either the compounds of formula I or the compounds of formula II wherein one or several nitrogen atoms are oxidized to the so-called N-oxide.

It will be appreciated that either the compounds of formula I or the compounds of formula II may have metal binding, chelating, complex forming properties and therefore may exist as metal complexes or metal chelates. Such metalated derivatives of the compounds of formula I are intended to be included within the scope of the present invention.

Some of the compounds of either the compounds of formula I or the compounds of formula II may also exist in their tautomeric form. Such forms although not explicitly indicated in the above formula are intended to be included within the scope of the present invention.

The term "alkyl" as used herein it does refer to any linear, branched, or cyclic hydrocarbon in which all carbon-carbon bonds are single bonds. Alkyl chains may optionally be substituted by heteroatoms.

The term "alkenyl" and "unsubstituted alkenyl" are used interchangeably herein and refer to any linear, branched, or cyclic alkyl with at least one carbon-carbon double bond.

Furthermore, the term "alkynyl" as used herein it does refer to any linear, branched, or cyclic alkyl or alkenyl with at least one carbon-carbon triple bond.

The term "aryl" as used herein it does refer to any aromatic cyclic alkenyl or alkynyl, being as a group or part of a group is phenyl or naphthalenyl, each optionally substituted with one, two or three substituents selected from halo, hydroxy, nitro, cyano, carboxyl, C₁₋₆alkyl, C₁₋₆alkoxy, C₁₋₆₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy. Preferred aryl groups are phenyl and naphtyl. The term "alkaryl" is employed where an aryl is covalently bound to an alkyl, alkenyl, or alkynyl.

The term "substituted" as used herein refers to a replacement of an atom or chemical group (e.g., H, NH₂, or OH) with a functional group, and particularly contemplated functional groups include nucleophilic groups (e.g., -NH₂, -OH, -SH, -NC, etc.), electrophilic groups (e.g., C(O)OR, C(X)OH, etc.), polar groups (e.g., -OH), non-polar groups (e.g., aryl, alkyl, alkenyl, alkynyl, etc.), ionic groups (e.g., -NH₃⁺), and halogens (e.g., -F, -CI), and all chemically reasonable combinations thereof. Thus, the term "functional group" and the term "substituent" are used interchangeably herein and refer to nucleophilic groups (e.g., -NH₂,-OH, -SH, -NC, -CN, etc.), electrophilic groups (e.g., C(O)OR, C(X)OH, C(Halogen)OR, etc.), polar groups (e.g., -OH), non-polar groups (e.g., aryl, alkyl, alkenyl, alkynyl, etc.), ionic groups (e.g., -NH₃⁺), and halogens.

The term "optionally substituted" when referring to alkyl, alkenyl, alkynyl, cycloalkyl, cycloalkenyl, aryl and heterocycle is intended to cover groups having oxo, ethylenedioxy, alkanoyloxy, alkoxy, alkylthio, carboxyl, halogen, thienyl, acetyl, 1-oxopropyl, 2-oxopropyl, 2-oxobutyl, 3-oxobutyl, 3-oxopentyl, 4-oxopentyl, 4-oxohexyl, 5-oxohexyl, ethylenedioxymethyl, 1,1-ethylenedioxyethyl, 2,2-ethylenedioxyethyl, 1,1-ethylenedioxypropyl, 2,2-ethylenedioxypropyl, 3,3-ethylenedioxypropyl, 1,1-ethylenedioxybutyl, 2,2-ethylenedioxybutyl, 3,3-ethylenedioxybutyl, 4,4-ethylenedioxybutyl, 3,3-ethylenedioxypentyl, 4,4-ethylenedioxyhexyl, 5,5-ethylenedioxyhexyl, acetyloxymethyl, 2-acetyloxyethyl, 3-acetyloxypropyl, 3-acetyloxybutyl, 4-acetyloxybutyl, 3-propionyloxybutyl, 3-butyryloxybutyl, 3-valeryloxypentyl, 3-hexanoyloxyhexyl, 4-acetyloxypentyl, 5-acetyloxypentyl, 4-acetyloxyhexyl, 5-acetyloxyhexyl, 6-acetyloxyhexyl, methoxymethyl, ethoxymethyl, propoxymethyl, butoxymethyl, pentyloxymethyl, hexyloxymethyl, 1-methoxyethyl, 2-methoxyethyl, 2-ethoxyethyl, 2-propoxyethyl, 2-butoxyethyl, 2-pentyloxyethyl, 2-hexyloxyethyl, 3-methoxypropyl, 3-ethoxypropyl, 2-methoxybutyl, 4-ethoxybutyl, 3-methoxypentyl, 5-ethoxypentyl, 4-methoxyhexyl, 6-ethoxyhexyl, methylthiomethyl, ethylthiomethyl, propylthiomethyl, butylthiomethyl, pentylthiomethyl, hexylthiomethyl, 1-methylthioethyl, 2-methylthioethyl, 2-ethylthioethyl, 2-methylthiopropyl, 3-methylthiopropyl, 3-ethylthiobutyl, 4-butylthiobutyl, 5-methylthiopentyl, 6-ethylthiohexyl, carboxymethyl, 1-carboxyethyl, 2-carboxyethyl, 2-carboxypropyl, 3-carboxypropyl, 4-carboxybutyl, 5-carboxypentyl, 6-carboxyhexyl, fluoromethyl, bromomethyl, chloromethyl, iodomethyl, 2-chloroethyl, 2-bromopropyl, 3-iodopropyl, 4-fluorobutyl, 5-chloropentyl, 6-bromohexyl, 2-thienylmethyl, 1-(2-thienyl)ethyl, 2-(2-thienyl)ethyl and the like.

The term "amino acid" refers to any of a class of organic compounds that contains at least one amino group, -NH-, and one carboxyl group, -COOH. These compounds can be the natural aminoacids present in peptides or can contain any substitution in the amino group, in the carboxyl group or in the side chain. They can also present different chirality of the peptidic natural aminoacids or can have different backbone, linear or cyclic, but must present, as said, at least one amino group and one carboxyl group. Amino acids can incorporate functional or protecting groups, such as those known for those skilled in the art. Preferred amino acids include, but are not limited to, alanine, valine, leucine and isoleucine.

As for the reaction conditions for the Reaction II-I, this process is preferably carried out at the following conditions, independently one of each other:
- the temperature ranges from 18 to 100 °C, preferably from 20 to 100°C;
- the reaction time ranges from 1 minute to 600 h;
- the concentration of compound of formula (II) or a derivative thereof as defined above ranges from 0.1 mM to 500 M;
- the amount of enzyme having deaminase activity ranges from 0.001 to 10000 mg/ml, preferably from 0.001 to 1000 mg/ml, in terms of concentration, or alternatively, the amount of enzyme having deaminase activity ranges from 0.001 to 10000 AU/micromol substrate, preferably from 0.001 to 100 AU/micromol substrate, more preferably from 0.001 to 25 AU/micromol substrate, in terms of enzyme activity.

In another embodiment, the reaction medium is aqueous optionally also containing up to 40%, preferably up to 20% and more preferably up to 15% of a suitable organic solvent. Preferably, said organic solvent is a polar aprotic solvent, preferably selected from tetrahydrofuran, 2-methyltetrahydrofuran, acetonitrile, acetone, cyclopentyl methyl ether, methyl ethyl ketone, methyl isobutyl ketone, dimethylamide and dimethylformamide.

The process according to present invention may also include isolation and/or purification steps of the NA produced by standard operation means selected from chromatography, precipitation, filtration, concentration and crystallization.

The present invention will be further described by means of examples which do not intend to limit the scope of the instant invention. Comparative examples are also provided.

### EXAMPLES

### Comparative Example 1: Deamination of cytidine to uridine

A 100 mM solution of the substrate (495 ul) in 100 mM phosphate buffer at pH 7 was mixed with 50 ul of deaminase enzyme extract containing >50 AU in phosphate buffer (AU: Activity unit, wherein 1AU is defined as the amount of enzyme capable of converting 1 micromol of substrate into product in a minute) . The reaction was performed at 37°C during 5 minutes and stopped with HCl. Then, the crude reaction was filtered through a 10 KDa membrane, and a portion was diluted and analyzed by HPLC. The expected uridine product was detected by UV-DAD (ultraviolet-diode array detection), in quantitative conversion (>99%).

### Comparative Example 2: Deamination of cytidine 5'-monophosphate to uridine 5'-monophosphate

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 minutes and stopped with HCl. Then, the crude reaction was filtered through a 10 KDa membrane, and a portion was diluted and analyzed by HPLC. The expected uridine 5'-monophosphate product was not detected by UV-DAD (ultraviolet-diode array detection), due to no conversion of the substrate into the final product (0 %).

### Comparative Example 3: Deamination of 2'-deoxycytidine to 2'-deoxyuridine

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 minutes and stopped with HCl. Then, the crude reaction was filtered through a 10 KDa membrane, and a portion was diluted and analyzed by HPLC. The expected uridine product was detected by UV-DAD (ultraviolet-diode array detection), in quantitative conversion (>99%).

### Comparative Example 4: Deamination of 2'-deoxycytidine 5'-monophosphate to 2'-deoxyuridine 5'-monophosphate

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 minutes and stopped with HCl. Then, the crude reaction was filtered through a 10 KDa membrane, and a portion was diluted and analyzed by HPLC. The expected 2'-deoxyuridine 5'-monophosphate product was not detected by UV-DAD (ultraviolet-diode array detection), due to no conversion of the substrate into the final product (0 %).

### Comparative Example 5: Deamination of cytidine 5'-triphosphate to uridine 5'-triphosphate

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 minutes and stopped with HCl. Then, the crude reaction was filtered through a 10 KDa membrane, and a portion was diluted and analyzed by HPLC. The expected uridine 5'-triphosphate product was not detected by UV-DAD (ultraviolet-diode array detection), due to no conversion of the substrate into the final product (0 %).

### Comparative Example 6: Deamination of cytarabine (cytosine arabinoside) to uridine arabinoside

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 minutes and stopped with HCl. Then, the crude reaction was filtered through a 10 KDa membrane, and a portion was diluted and analyzed by HPLC. The expected product was detected by UV-DAD (ultraviolet-diode array detection), in quantitative conversion (>99%).

### Comparative Example 7: Deamination of cytarabine 5'-monophosphate (cytosine arabinoside 5'-monophosphate) to uridine arabinoside 5'-monophosphate

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 minutes and stopped with HCl. Then, the crude reaction was filtered through a 10 KDa membrane, and a portion was diluted and analyzed by HPLC. The expected product was not detected by UV-DAD (ultraviolet-diode array detection), due to no conversion of the substrate into the final product (0 %).

Example 6: Deamination of isopropyl (((5-(4-amino-2-oxopyrimidin-1(2H)-yl)-3,4-dihydroxytetrahydrofuran-2-yl)methoxy)(phenoxy)phosphoryl)-L-alaninate to isopropyl (((5-(4-amino-2-oxopyrimidin-1(2H)-yl)-3,4-dihydroxytetrahydrofuran-2-yl)methoxy)(phenoxy)phosphoryl)-L-alaninate

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 hours and stopped with HCl. Then, the crude reaction was filtered through a 10 KDa membrane, and a portion was diluted and analyzed by HPLC. The expected product was detected by UV-DAD (ultraviolet-diode array detection), in quantitative conversion (>99%).

Example 7: Deamination of tert-butyl ((2R,4R,5S)-2-((5-(4-amino-2-oxopyrimidin-1(2H)-yl)-3,4-dihydroxytetrahydrofuran-2-yl)methoxy)-2-oxido-4-phenyl-1,3,2-dioxaphosphinan-5-yl)carbamate to tert-butyl ((2R,4R,5S)-2-((5-(2,4-dioxo-3,4-dihydropyrimidin-1(2H)-yl)-3,4-dihydroxytetrahydrofuran-2-yl)methoxy)-2-oxido-4-phenyl-1,3,2-dioxaphosphinan-5-yl)carbamate

A 100 mM solution of the substrate (495 ul) in 100 mM phosphate buffer at pH 7 was mixed with 50 ul of deaminase enzyme extract containing >50 AU in phosphate buffer. The reaction was performed at 37°C during 5 hours and stopped with HCl. Then, the crude reaction was filtered through a 10 KDa membrane, and a portion was diluted and analyzed by HPLC. The expected product was detected by UV-DAD (ultraviolet-diode array detection), in quantitative conversion (>99%).

As it has been demonstrated in comparative examples, the deamination of nucleosides using nucleoside deaminases works at quantitative conversion (cytidine (comparative example 1), 2'-deoxycytidine (comparative example 3) and cytarabine (comparative example 6)), due to the fact that nucleosides bearing no substitution at OH-5' are the natural substrates for these enzymes.

On the other hand, no transformation is obtained when the substrate is the corresponding nucleotide (cytidine 5'-monophosphate (comparative example 2), 2'-deoxycytidine 5'-monophosphate (comparative example 4), cytidine 5'-triphosphate (comparative example 5) and cytarabine 5'-monophosphate (comparative example 7)), as it is expected, because the molecules bearing a phosphate substitution at OH-5' are not recognized by these nucleoside deaminase enzymes.

However, inventors have surprisingly found that when the functionality of the nucleoside at OH-5' is in the form of substituted organophosphorous nucleoside, excluding natural nucleotides, deamination reaction takes place at conversions and yields similar to those obtained in the natural non-OH-5' substituted nucleosides. This is a teaching away from what is reported in literature, since some authors have disclosed that nucleosidic substrates incorporating bulky substituents exhibit difficult fitting into the active site of the nucleoside deaminase enzymes, and in some cases, they are even inhibitors of these type of enzymes (Faivre-Nitschke, S.E. et al. 1999, A prokaryotic-type cytidine deaminase from Arabidopsis thaliana, Eur. J. Biochem. 263, 896-903).

## Claims

1. Process for preparing a compound of formula (I) according to the following reaction catalyzed by a nucleoside deaminase: 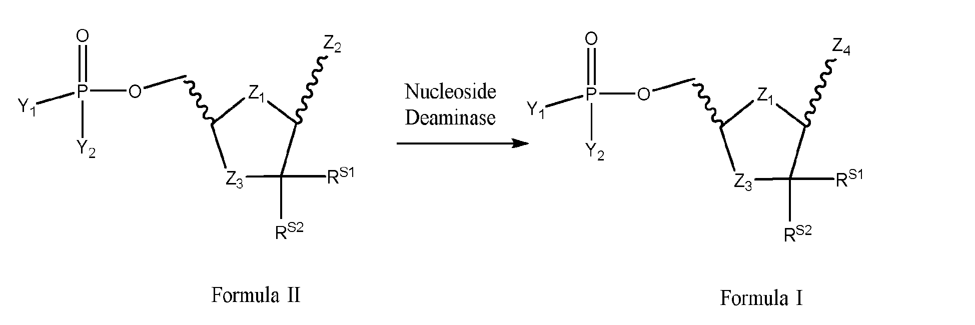 wherein
Z₁ is selected from O, CH₂, S and NH;
Z₃ is selected, independently of Z₁, from O, C(R^{S3}R^{S4}), S(R^{S3}R^{S4}), S(R^{S3}) and N(R^{S3});
Z₂ is selected from: 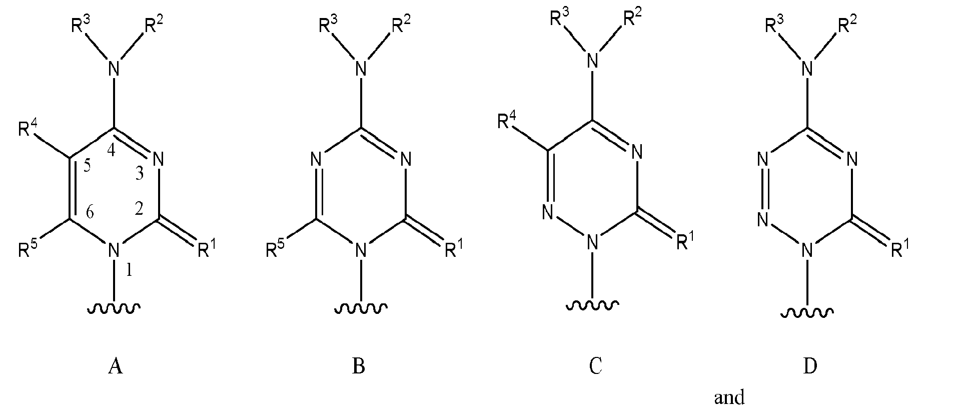
Z₄ is selected from: 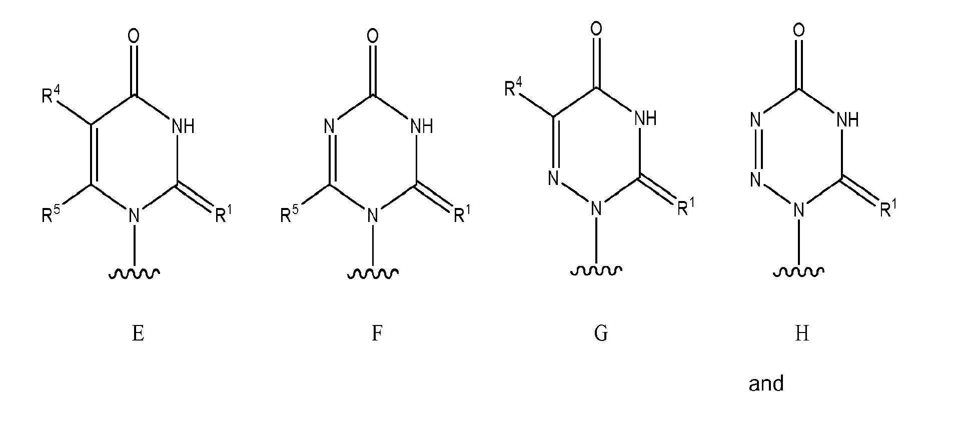
R¹ is selected from O, CH₂, alkyl, S and NH;
R² is selected from hydrogen; methyl; an optionally substituted alkyl chain; an optionally substituted alkenyl chain; an optionally substituted alkynyl chain; trihaloalkyl; NR⁶R⁷; CN; COR⁶; CONR⁶R⁷; CO₂R⁶; C(S)R⁶; C(S)OR⁶; NHCONR⁶R⁷; NHCO₂R⁶; NHC(S)OR⁶; SO₂R⁶; SO₂NR⁶R⁷; an optionally substituted aryl linked to N-4 by an optionally substituted alkyl, alkenyl or alkynyl chain; and an optionally substituted heterocycle linked to N-4 by an optionally substituted alkyl, alkenyl or alkynyl chain;
R³ is selected, independently of R², from hydrogen; methyl; an optionally substituted alkyl chain; an optionally substituted alkenyl chain; an optionally substituted alkynyl chain; trihaloalkyl; NR⁶R⁷; CN; COR⁶; CONR⁶R⁷; CO₂R⁶; C(S)R⁶; C(S)OR⁶; NHCONR⁶R⁷; NHCO₂R⁶; NHC(S)OR⁶; SO₂R⁶; SO₂NR⁶R⁷; an optionally substituted aryl linked to N-4 by an optionally substituted alkyl, alkenyl or alkynyl chain; and an optionally substituted heterocycle linked to N-4 by an optionally substituted alkyl, alkenyl or alkynyl chain;
providing that at least one of R² and R³ is H;
R⁴ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶;NHCONR⁶R⁷; NHCOR⁶; NR⁶CO₂R⁷; NHCO₂R⁶; NHC(S)OR⁶; SO₂NR⁶R⁷; an optionally substituted aryl linked to C-5 by an optionally substituted alkyl, alkenyl or alkynyl chain; and an optionally substituted heterocycle linked to C-5 by an optionally substituted alkyl, alkenyl or alkynyl chain;
R⁵ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶;NHCONR⁶R⁷; NHCOR⁶; NR⁶CO₂R⁷; NHCO₂R⁶; NHC(S)OR⁶; SO₂NR⁶R⁷; an optionally substituted aryl linked to C-6 by an optionally substituted alkyl, alkenyl or alkynyl chain; and an optionally substituted heterocycle linked to C-6 by an optionally substituted alkyl, alkenyl or alkynyl chain;
R⁶ and R⁷ are selected, independently of each other, from hydrogen; an optionally substituted alkyl chain; an optionally substituted alkenyl chain; an optionally substituted alkynyl chain; an optionally substituted heterocycle; and an optionally substituted aryl, preferably phenyl or naphtyl;
R^{S1} is selected, independently of R^{S2}, from hydrogen; halogen, preferably F; methyl; OH; NH₂; SH; N₃; an optionally substituted alkyl chain; an optionally substituted alkenyl chain; an optionally substituted alkynyl chain; trihaloalkyl, OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S2} is selected, independently of R^{S1}, from hydrogen; halogen, preferably F; methyl; OH; NH₂; SH; N₃; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S3} is selected, independently of R^{S4}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; and an optionally substituted aryl; an optionally substituted heterocycle; OR⁶; OCONR⁶R⁷; OCOR⁶; OCO₂R⁶; and OC(S)OR⁶;
R^{S4} is selected, independently of R^{S3}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; an optionally substituted aryl; an optionally substituted heterocycle; OR⁶; OCONR⁶R⁷; OCOR⁶; OCO₂R⁶; and OC(S)OR⁶;
Y₁ is selected, independently of Y₂, from hydrogen; OR⁸; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O-*naphtyl; an ether of an optionally substituted heterocycle; and an amino acid, preferably alanine, valine, leucine or isoleucine, either in the free form or protected by a suitable functional group;
Y₂ is selected, independently of Y₁, from hydrogen; OH; OR⁸; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O-*naphtyl; an ether of an optionally substituted heterocycle; an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; and an optionally substituted heterocycle optionally linked to P through O or N atoms; aryl, preferably phenyl or naphthyl;
and wherein the compound of formula (I) or formula (II) can also be a derivative thereof, being said derivative an acceptable pharmaceutically salt thereof; an ester, amide, carbamate or phosphate thereof; an addition salt thereof; a quaternary ammonium salt thereof; or an N-oxide thereof.

2. Process for preparing a compound of formula (I), according to claim 1, wherein
Z₁ is selected from O and CH₂, more preferably O;
Z₃ is selected, independently of Z₁, from O and C(R^{S3}R^{S4}), more preferably C(R^{S3}R^{S4});
Z₂ is selected from 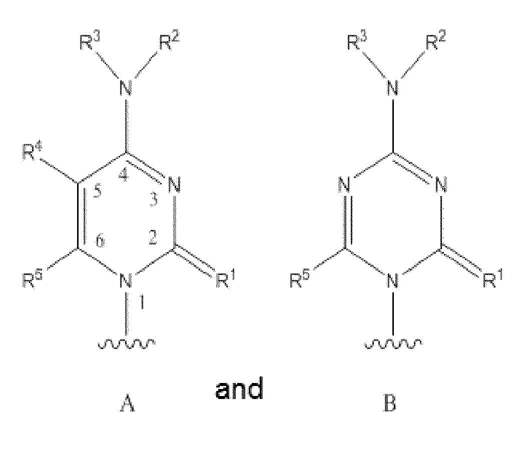
R¹ is O;
R² is selected from H; methyl; an optionally substituted alkyl chain; COR⁶, CONR⁶R⁷, CO₂R⁶, and C(S)OR⁶; more preferably selected from H; COR⁶; CONR⁶R⁷; CO₂R⁶; and even more preferably R² is COR⁶, CONR⁶R⁷, or CO₂R⁶;
R³ is selected, independently of R², from H; methyl; an optionally substituted alkyl chain; COR⁶; CONR⁶R⁷; CO₂R⁶; C(S)OR⁶; more preferably R³ is H;
providing that at least one of R² and R³ is H;
R⁴ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R⁵ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F, methyl; OH; OR⁶; NR⁶R⁷; OCONR⁶R⁷; OCOR⁶; OCO₂R⁶; and OC(S)OR⁶;
R^{S2} is selected, independently of R^{S1}, from hydrogen; halogen, preferably F, methyl; OH; OR⁶; NR⁶R⁷; OCONR⁶R⁷; OCOR⁶; OCO₂R⁶; and OC(S)OR⁶;
R^{S3} is selected, independently of R^{S4}, from hydrogen; OH; OR⁶; OCONR⁶R⁷; OCOR⁶; OCO₂R⁶; OC(S)OR⁶; and halogen, preferably F;
R^{S4} is selected, independently of R^{S3}, from hydrogen; OH; OR⁶; OCONR⁶R⁷; OCOR⁶; OCO₂R⁶; OC(S)OR⁶; and halogen, preferably F;
Y₁ is selected, independently of Y₂, from hydrogen; OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from hydrogen; OH; OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

3. Process for preparing a compound of formula (I), according to claim 1, wherein
Z₁ is O;
Z₃ is C(R^{S3}R^{S4});
Z₂ is selected from 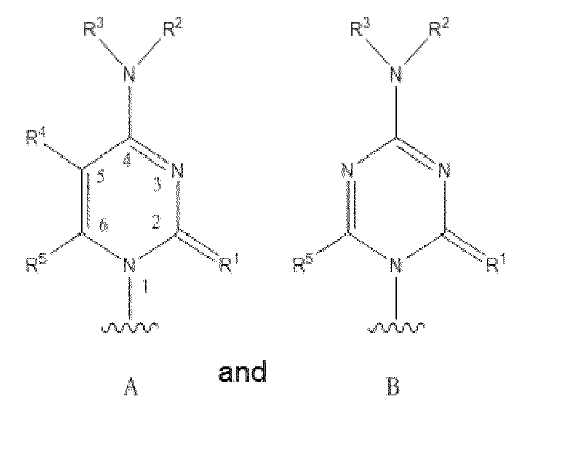
R¹ is O;
R² is selected from H; methyl; COR⁶; CONR⁶R⁷; and CO₂R⁶;
R³ is selected, independently of R², from H; methyl; COR⁶; CONR⁶R⁷; CO₂R⁶; and C(S)OR⁶; more preferably R³ is COR⁶, CONR⁶R⁷ or CO₂R⁶;
providing that at least one of R² and R³ is H;
R⁴ is selected from H; OH; halogen, preferably F; and trihaloalkyl;
R⁵ is selected from H; OH; halogen; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F; methyl; OH; OR⁶; OCONR⁶R⁷; OCOR⁶; and OCO₂R⁶;
R^{S2} is selected, independently of R^{S1}, from hydrogen; halogen preferably F; methyl; OH; OR⁶; OCONR⁶R⁷; OCOR⁶; OCO₂R⁶;
R^{S3} is selected, independently of R^{S4}, from hydrogen; OH; OR⁶; OCONR⁶R⁷; OCOR⁶; OCO₂R⁶; OC(S)OR⁶; halogen, preferably F;
R^{S4} is selected, independently of R^{S3}, from hydrogen; OH; OR⁶; OCONR⁶R⁷; OCOR⁶; OCO₂R⁶; OC(S)OR⁶; halogen, preferably F;
Y₁ is selected, independently of Y₂, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from hydrogen; OH; OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

4. Process for preparing a compound of formula (I), according to claim 1, wherein
Z₁ is O;
Z₃ is C(R^{S3}R^{S4}) wherein R^{S3} is H or OH and wherein R^{S4} is, independently of R^{S3}, H or OH;
Z₂ is 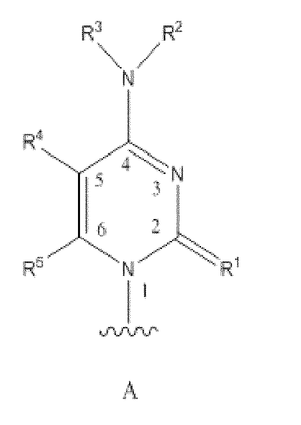
R¹ is O;
R² is H;
R³ is H;
R⁴ is H or halogen, preferably F;
R⁵ is H;
R^{S1} is selected, independently of R^{S2}, from hydrogen; halogen, preferably F; methyl; and OH; R^{S2} is selected, independently of R^{S1}, from hydrogen; halogen, preferably F; methyl; and OH; Y₁ is selected, independently of Y₂, from OR⁸; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; NHCONR⁶R⁷; NHCOR⁶; NR⁶CO₂R⁷; NHCO₂R⁶; NHC(S)OR⁶; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from methyl; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl.

5. Process for preparing a compound of formula (I), according to claim 4, wherein
Y₁ is selected, independently of Y₂, from OR⁸; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from an ether of an optionally substituted aryl, preferably *O*-phenyl or *O*-naphtyl; and an amino acid, preferably alanine, valine, leucine or isoleucine, either in the free form or protected by a suitable functional group.

6. Process for preparing a compound of formula (I), according to claim 4, wherein
Y₁ is selected, independently of Y₂, from an ether of an optionally substituted aryl, preferably *O*-phenyl; and an amino acid, preferably alanine, valine, leucine or isoleucine, either in the free form or protected by a suitable functional group;
Y₂ is selected, independently of Y₁, from an ether of an optionally substituted aryl, preferably *O*-phenyl; and an amino acid, preferably alanine, valine, leucine or isoleucine, either in the free form or protected by a suitable functional group.

7. Process, according to any of the preceding claims, wherein the nucleoside deaminase is a cytidine deaminase.

8. Process, according to any of the preceding claims, wherein said process is carried out at a temperature ranging from 18 to 100 °C, preferably from 20 to 100°C.

9. Process, according to any of the preceding claims, wherein the reaction time for said process ranges from 1 minute to 600 h.

10. Process, according to any of the preceding claims, wherein the concentration of compound of formula (II) or a derivative thereof ranges from 0.1 mM to 500 M.

11. Process, according to any of the preceding claims, wherein the amount of enzyme having deaminase activity ranges from 0.001 to 10000 mg/ml, preferably from 0.001 to 1000 mg/ml.

12. Process, according to any of the preceding claims, wherein the amount of enzyme having deaminase activity ranges from 0.001 to 10000 AU/micromol substrate, preferably from 0.001 to 100 AU/micromol substrate, more preferably from 0.001 to 25 AU/micromol substrate.

13. Process, according to any of the preceding claims, wherein the reaction medium is aqueous optionally also containing up to 40%, preferably up to 20% and more preferably up to 15% of a suitable organic solvent.

14. Process, according to claim 13, wherein said organic solvent is a polar aprotic solvent, preferably selected from tetrahydrofuran, 2-methyltetrahydrofuran, acetonitrile, acetone, cyclopentyl methyl ether, methyl ethyl ketone, methyl isobutyl ketone, dimethylamide and dimethylformamide.
